# EUROPEAN PATENT SPECIFICATION

(11) **EP 1 475 036 B1**
(45) Date of publication and mention of the grant of the patent: **27.07.2005**
(21) Application number: 03101301.4
(22) Date of filing: 09.05.2003
(51) Int. Cl.: A61B 5/0215, A61M 25/01

(54) **Sensor guide wire**
Führungsdrahteinheit mir Sensor
Fil de guidage avec capteur

(43) Date of publication of application: 10.11.2004
(73) Proprietor: RADI MEDICAL SYSTEMS AB, 754 50 Uppsala (SE)
(72) Inventor: Von Malmborg, Pär, 755 97, UPPSALA (SE); Smith, Leif, 756 52, UPPSALA (SE); Akerfeldt, Dan, 755 92, UPPSALA (SE); Hammarström, Ola, 540 17 Lerdala (SE)
(74) Representative: Holmberg, Nils Anders Patrik

(56) References cited:
- EP-A- 1 108 441
- US-A- 5 226 423
- US-A- 6 112 598
- US-A- 6 142 958

## Description

### Field of the invention

The present invention relates to a core wire and a sensor guide wire assembly according to the preambles of the independent claims.

The invention relates generally to sensors mounted on guide wires for intravascular measurements of physiological variables in a living body, in particular to the design of such guide wires, and more particularly to the design of the core wires in such guide wires.

### Background of the invention

Sensor and guide wire assemblies in which a sensor, adapted for measurements of physiological variables in a living body, such as blood pressure and temperature, is mounted at the distal end of a guide wire are known.
In US-5,226,423, which is assigned to the present applicant, is disclosed one example of such a sensor and guide wire assembly, in which a sensor guide comprises a sensor element, an electronic unit, a signal transmitting cable connecting the sensor element to the electronic unit, a flexible tube having the cable and the sensor element disposed therein, a solid metal wire, which is disposed inside the sensor guide and extends along the entire length of the sensor guide inside the flexible tube, and a coil attached to the distal end of the solid metal wire. The solid metal wire, also known as a core wire, has been divided into a plurality of sections, and near the distal end of the sensor guide, the sensor element is positioned in one of these sections having an enlarged thickness.

A sensor and guide wire assembly, similar to that of US-5,226,423 and also assigned to the present applicant, is shown in US-6,142,958. According to US-6,142,958, the core wire extends out from the distal end of the flexible tube, and a first coil is provided between the section having an enlarged thickness and the distal end of the flexible tube, while a second coil is attached to the distal end of the section having an enlarged thickness.

According to US-5,226,423 and US-6,142,958 each of the plurality of core wire sections has a different thickness and thereby a different flexibility. Apparently, a large flexibility of the sensor guide is advantageous in that it allows the sensor guide to be introduced into small and tortuous vessels. It should, however, also be recognized that if the core wire being too flexible, it would be impossible to push the sensor guide forward into the vessels, i.e. the sensor guide must possess a certain "pushability".

Furthermore, the sensor guide wire must be able to withstand the mechanical stress exerted on the core wire especially in sharp vessel bends. In US-5,226,423 and US-6,142,958 no figures are given for the dimensions (e.g. the diameters) of the different sections of the core wire, but a sensor guide of this type which is manufactured and sold by the applicant of the present application under the registered trademark PRESSUREWIRE® has a portion adjacent the proximal end of the enlarged section, which contains the sensor element, with a diameter of 130 µm and a portion adjacent the distal end of the enlarged section with a diameter of 90 µm.

Although this design of the known sensor guide wire has proven to work very well it can be improved.

### Summary of the invention

The object of the present invention is therefore to provide a core wire to be used in a sensor and guide wire assembly with such design and such dimensions that the sensor and guide wire assembly is provided with improved mechanical properties regarding flexibility and strength.

This object is achieved with a core wire according to the present invention.

According to the present invention it has been found that the mechanical strength of a sensor guide wire of the type described above is surprisingly sensitive to the dimensions of the different core wire portions distally and proximally of the enlarged portion accommodating the sensor element. In particular, tests have shown that the portion of the core wire that is adjacent the proximal end of this enlarged portion should have the same diameter as the portion of the core wire that is adjacent the distal end of the enlarged portion.

By following these findings, it has even more surprisingly been found that the diameter of the portion proximally of the enlarged portion can be decreased without deteriorate the strength of the core wire as a whole. In fact, extensive tests have shown that by reducing the diameter of the portion proximally of the enlarged portion with 20 µm, from 130 µm to 110 µm, and by increasing the diameter of the portion distally of the enlarged portion with 20 µm, from 90 µm to 110 µm, the strength of the sensor guide wire is enhanced by a factor of seven (7).

### Brief description of the drawings

Fig. 1 is a cross-section of a sensor and guide wire assembly according to a conventional design.
Fig. 2 shows schematically a core wire according to a first preferred embodiment of the present invention.
Fig. 3 illustrates the behaviour in sharp bends of a core wire provided with a stiff portion.
Fig. 4 illustrates the behaviour in sharp bends of a wire without a stiff portion.
Fig. 5 shows schematically a core wire according to a second preferred embodiment of the present invention.
Fig. 6a is a cross-section of a sensor and guide wire with a core wire according to the present invention.
Fig. 6b is a cross-section of the enlarged section of the core wire, with a sensor element disposed therein.

### Detailed description of preferred embodiments

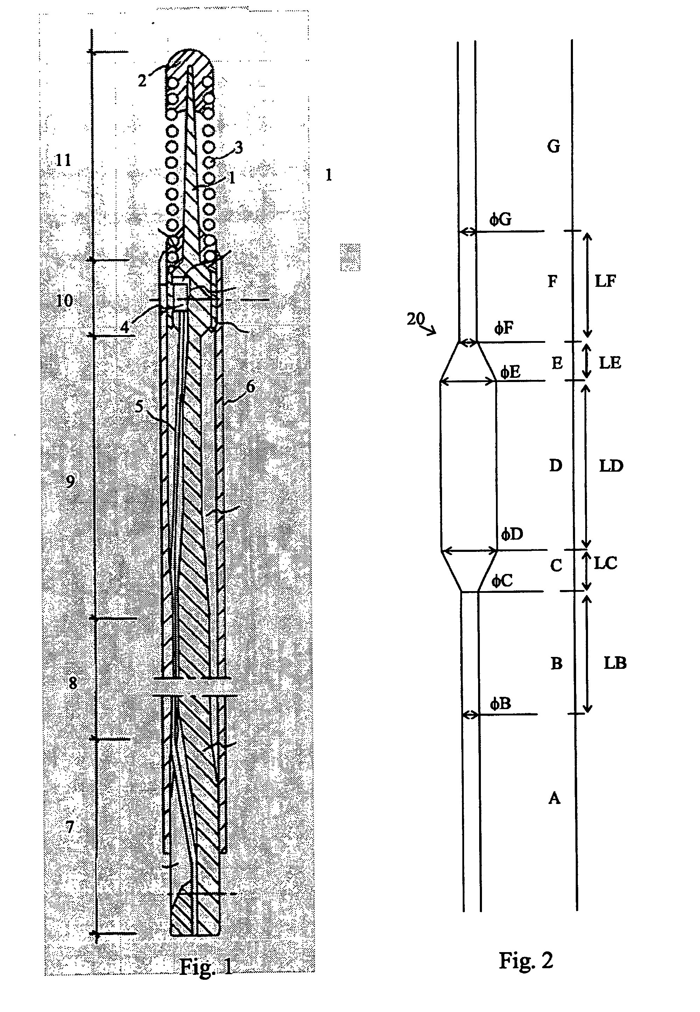

Now with reference to Figure 1, a sensor guide wire according to the above-identified prior art comprises a core wire 1 whose distal end is provided with a dome-shaped tip 2, a coil 3 attached to the dome-shaped tip 2 and to an enlarged portion of the core wire 1, a sensor element 4 to which at least one signal transmitting cable 5 is connected, and an outer tube 6 which at least partly encloses the core wire 1.

The sensor and guide wire assembly of figure 1 has been divided into five sections, 7-11, where section 11 is the most distal section of the assembly, i.e. the section that is going to be inserted farthest into a vessel, and section 7 is the most proximal section. In a preferred embodiment according to US-5,226,423, section 7 is about 10-100 mm, section 8 is about 1000-2000 mm, section 9 is about 200-400 mm, section 10 is about 1-5 mm, and section 11 is about 10-50 mm. The diameter of the sensor and guide wire assembly varies between 0.25-2 mm; for use in coronary arteries, the diameter is normally 0.35 mm.

The core wire 1, which extends along the entire length of the sensor and guide wire assembly, is preferably made from a metal, such as stainless steel, or a superelastic metal, e.g. Nitinol®. As should be apparent from figure 1 and as is generally known in the art, the mechanical properties (e.g. flexibility and strength) of the sensor guide wire will mainly be determined by the material, design and dimensions of the core wire. In order to enhance the possibility to control the mechanical characteristics of a sensor guide wire, the core wire in each of the sections 7-11 can therefore be given a separate thickness. As was mentioned above, the US-5,226,423 discloses a sensor guide wire with a core wire having a plurality of sections where each section has a different thickness and thereby a different flexibility.

Figure 2 illustrates schematically a core wire 20, the dimensions of which are the subject of the present invention. The core wire 20 has been divided into seven sections or portions, A-G, where section A is the most proximal section and section G is the most distal section.

The sections relevant for the present application are, where appropriate, characterized by a length and a diameter, where the diameter is taken at proximal beginning of the section in question. So has, for example, section B a diameter dB and a length LB, section C a diameter dC and a length LC, section D a length LD and a diameter dD, etc. Please observe that in the drawings the diameters are indicated by a circle with a crossing line.

The core wire 20 illustrated in figure 2 is intended to be used in a sensor and guide wire assembly like the one illustrated in figure 1, and is in figure 2 shown in a state before assembling and mounting of parts such as the sensor element, the signal transmitting cable(s), the dome-shaped tip and the coil. Especially the enlarged portion, which according to figure 2 consists of sections C, D and E, has not yet been provided with a recess for reception of a sensor element. Such a recess or depression in the enlarged portion can be made by spark machining, while the enlarged portion itself can be made by removing material from a metal wire having the nominal diameter of the enlarged portion so as to form the smaller diameter portions of the core wire extending distally and proximally of the enlarged portion.

The lengths and especially the diameters of the different sections A-G will be discussed in more detail below, but here it can be mentioned that section B is about 0-5 mm, section C about 0-1 mm, section D about 0-4 mm, section E about 0-1 mm, and section F about 0-30 mm. It should therefore be noted that the sections A-G of figure 2 have no direct relationship to the sections 7-11 of figure 1. Further, the subject of the present invention is the dimensions of the sections B-F in general and the diameters dB, dC, dF and dG in particular.

A comparison between the lengths of the sections 7-11 of figure 1 and the lengths of the sections B-F of figure 2 reveals that the present invention is directed to a restricted portion of a core wire in the vicinity of the enlarged portion that is going to accommodate a sensor element.

From a mechanical (i.e. bending) point of view, the enlarged portion of a core wire can be regarded as stiff, and the object of the present invention is to find suitable dimensions for the portions distally and proximally of this enlarged portion. A sensor guide wire having a core wire with such suitable dimensions should be stiff enough to be pushed forward in narrow and tortuous vessels and yet be flexible enough for manoeuvring into acute takeoffs. The core wire must, at the same time, have such strength so that the performance regarding ability to withstand breakage during operation is further improved.

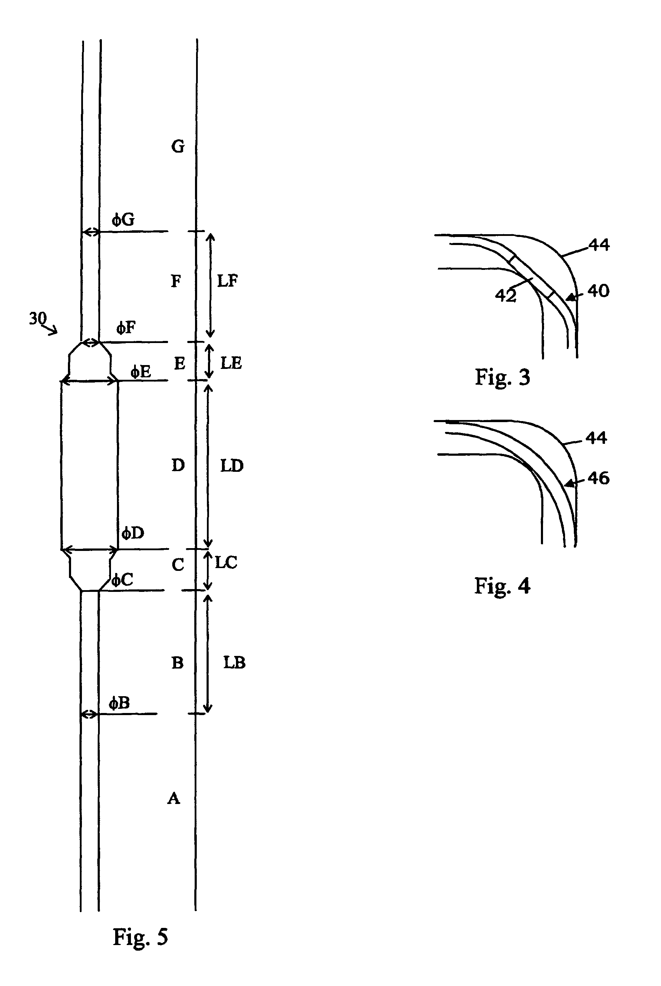

As was mentioned above, it has according to the present invention been found that the strength of a core wire having the general configuration shown in figure 2, with a stiff and relatively short enlarged portion, is surprisingly sensitive to the diameters of the portions proximally and distally of this enlarged portion. This fact can be qualitatively understood from an inspection of figure 3, which illustrates, in an experimental set-up, how a core wire 40, which includes a portion 42 that can be regarded as stiff, is manoeuvred through a sharp 90-degree bend 44. From the figure one can imagine that the portions distally and proximally of the stiff portion will experience a large strain when the stiff portion is pushed through the sharp bend. It may also be imagined that the strain is concentrated to rather restricted areas, something that increases the risk of having a break of the core wire in these restricted areas.

As a comparison, figure 4 illustrates, also in an experimental set-up, how a similar wire 46, which is not provided with a stiff portion, is manoeuvred through the same 90-degree bend 44. In the latter case, the strain exerted on the wire is distributed over large areas, and there are no obvious sites with increased risks of having a break. Here it should be emphasized that the situations illustrated in figures 3 and 4 are not intended to imitate situations known to actually prevail during introduction of a guide wire in a vessel. On the contrary, the situations illustrated in figure 3 and figure 4 should instead be regarded as part of the analysis leading to the present invention.

By means of experimental set-ups five different core wire designs were tested, but before presenting the results from these tests in detail it can be mentioned that in the tests the core wires consisted of solid metal wires, whose portions distally and proximally of the enlarged portion exhibited circular cross-sections, the diameters of which were varied. With these prerequisites, the conclusion from the tests is that the diameters of the portions proximally and distally of the enlarged portion should be the same. Here, one has, however, to remember that what actually was varied was the bending resistance, and if the portions proximally and distally of the enlarged portion would have consisted of different materials, with different mechanical properties, or of the same material but with different cross-section shapes, the corresponding conclusion would have been that the bending resistance of the portions proximally and distally of the enlarged portion should be the same. Another way to express this statement is that if the enlarged portion, which according to figure 2 consists of sections C, D and E, is removed and the adjoining portions, i.e. sections B and F, are connected to each other, the joint should be as smooth as possible, i.e. the bending resistance should exhibit a continuous behaviour.

As was mentioned above, the enlarged portion of a core wire can be regarded as stiff in comparison with the smaller dimension portions. This means that the specific shape of the enlarged portion does not significantly influence the overall mechanical characteristics of the core wire, which, in turn, implies that, for example, the tapered or conical portions (i.e. portions C and E in figure 2) of the enlarged portion can be divided into several conical or tapered portions.

Figure 5 illustrates a second preferred embodiment of a core wire 30, and a comparison between figure 2 and figure 5 shows that the design of the core wire 30 differs from the core wire 20 illustrated in figure 2 in that the tapered portions C and E of the core wire 20 of figure 2 have been further divided into additional portions in the core wire 30 of figure 5. However, for the purpose of the present application, also the core wire 30 of figure 5 can be regarded as having the same general design as the core wire 20 of figure 2, which is reflected by the fact that also the core wire 30 has been divided into seven sections or portions, A-G, where section A is the most proximal section and section G is the most distal section.

Thus, the present invention relates to a core wire for a sensor guide wire assembly for intravascular measurements of physiological variables in a living body. The core wire (20, 30) is provided with different longitudinal sections each having a section diameter (dA-dG), and comprises an enlarged portion, where a sensor is adapted to be arranged, including a predetermined number of sections (C, D, E), one or many distal sections (F, G) positioned distally said enlarged portion and one or many proximal sections (A, B) positioned proximally said enlarged portion, wherein at least one of the sections of said enlarged portion has a larger diameter compared to the diameters of the distal and proximal sections. The core wire is characterized in that the ratio between the diameters (dF, dC) adjacent the enlarged portion is close to 1, preferably between 0,95 and 1,05.
Alternatively this may be expressed as the relative absolute value difference between the diameters of the sections closest the enlarged portion is less than 5%.
Preferably the diameters of the core wire adjacent to the enlarged portion are equal, i.e. dC equals dF.
The sections E and C may be conically shaped as shown in figure 2 or the diameters in sections E and C may increase continuously step-wise as illustrated in figure 5.
A typical interval for dC and dF is in the interval 90-130 µm and the largest diameter of the enlarged section is 250 - 400 µm.

As illustrated in figure 2 and figure 5 the core wire is symmetrically shaped distally and proximally the enlarged portion. Specifically with regard to the diameters of the core wire, which are such that dC equals dF and dB equals dG, where dB and dG are taken at the same distance from the respective ends of the enlarged portion.
Here, the symmetry relates to restricted sections of the core wire distally and proximally the enlarged portion, i.e. in figures 2 and 5 the section B and section F, respectively. The relevant lengths (from a symmetry point of view) of these sections depend on the diameter of the enlarged portion of the core wire, but can be taken as less than 50 times the diameter of the enlarged portion.

Here it can be mentioned that although it is apparent from the example below that a core wire should be symmetrically shaped distally and proximally the enlarged portion in order to obtain maximal bending strength other considerations can be involved in the design of a sensor and guide wire assembly, of which a core wire is one part. If, for example, the core wire, despite the measures described herein, should break such that the sensor guide is held together only by one or several coils, it may be advantageous that this break is located distally of the enlarged portion rather than proximally of the enlarged portion. The reason for this is that it may be easier to successfully retract the sensor guide wire in one piece if the retraction is not obstructed by the comparatively large and stiff enlarged portion, which can get stuck in small and tortuous vessels. For a particular design of a sensor and guide wire assembly, a suitable choice may therefore be that the diameters adjacent the enlarged portion are equal, whereas the diameters at a distance proximally and distally of the enlarged portion differ slightly such that the proximal diameter is larger than the distal diameter. In figures 2 and 5, this means that dC=dF and dB>dG, where dB and dG are taken at the same distance from the respective ends of the enlarged portion. The diameter dB can be about 25% larger than the diameter dG. Here it should be noted that even for a sensor guide wire whose final design include a non-symmetrical core wire, according to the findings of the present invention, a symmetrical core wire would also in this case be the starting point for the design and construction work; and the present invention therefore provides an important contribution to the state of the art also in this case.

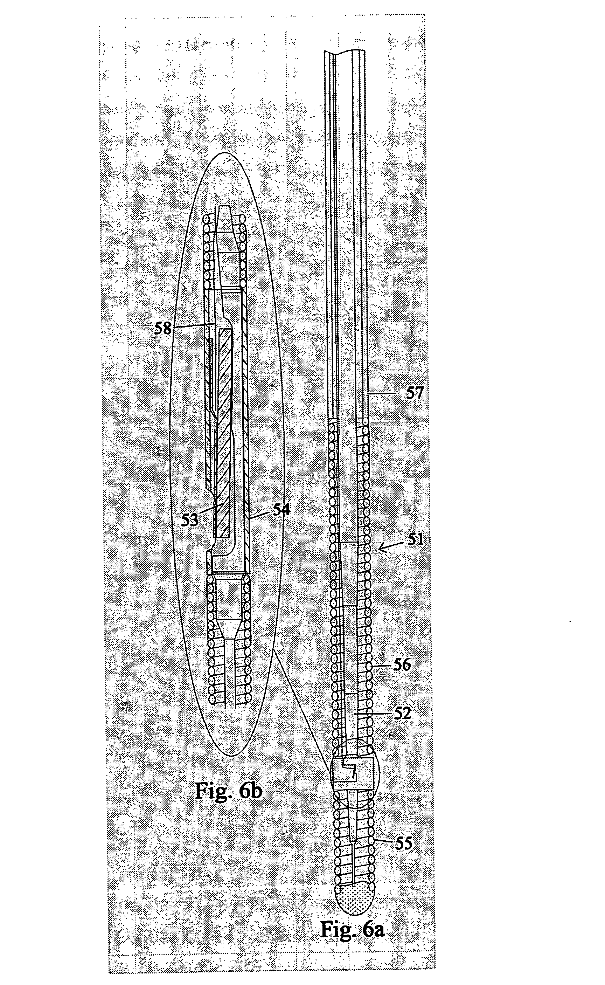

The present invention also relates to a sensor guide wire assembly for intravascular measurements of physiological variables in a living body. An example of such a sensor guide wire assembly is shown in figures 6a and 6b, where a sensor guide 51 comprises a core wire 52, which has been designed according to the teachings above and which extends along the entire length of the sensor guide 51 and has an enlarged portion in which a sensor element 53 is arranged. A jacket or sleeve 54 covers the sensor element 53 and at least a part of the enlarged portion of the core wire 52. At the distal end of this enlarged portion a first coil 55 is provided, while a second coil 56 is provided at the proximal end of the enlarged portion. The core wire 52 is partly disposed inside a flexible tube 57 such that the proximal end of the second coil 56 is attached to the distal end of the flexible tube 57. A sensor guide wire assembly comprises further an electronic unit (not shown in the figure) and a signal transmitting cable 58, which connects the sensor element 53 to the electronic unit and which is disposed inside the second coil 56 and the flexible tube 57.

Although the present invention has been described with reference to specific embodiments, also shown in the appended drawings, it will be apparent for those skilled in the art that many variations and modifications can be done within the scope of the invention as described in the specification and defined with reference to the claims below. In particular it can be said that the enlarged portion of the core wire, although in all cases being thick and stiff in comparison to its adjacent portions, can have a diameter that ranges from about three quarters of the diameter of the guide wire to the diameter of the guide wire, the last case being when no parts like coils, jackets or sleeves are provided around the enlarged portion.

Thus, in a more generalized description of the present invention a rigid portion of the guide wire is arranged adapted to be provided with a sensor. The rigid portion being stiff in comparison to its adjacent portions, a distal portion and a proximal portion. The distal portion and the proximal portion have equal bending resistances close to the rigid portion. This is achieved in accordance with the above described embodiment of the present invention where the ratio between the diameters close to the enlarged portion is close to 1. In the above described embodiment the guide wire preferably is made from one material. Alternatively, in another embodiment the equal bending resistance capability may also be achieved by appropriate choices of materials for the distal, rigid and proximal portions irrespectively of the diameters of the different portions. The alternative embodiment may be schematically illustrated as in figure 3 where the guide wire and the rigid portion are referred to as 40 and 42, respectively. Here, the rigid portion may be made from one material and the distal and proximal portions from another material. Alternatively, the distal and proximal portions may be made from different materials.

Furthermore, the above-mentioned embodiments of the core wire may have numerous different shapes and be made from a number of combinations of materials.
In one variant the distal sections are made from one material and the proximal section is made from another material. In one specifically advantageous embodiment the distal sections are made from nitinol and the proximal sections are made from stainless steel.
The distal sections may also be made from a combination of materials, preferably arranged such that a base material is covered by another material.

The required performance with regard to bending resistance may be achieved by heat-treatment of the core wire.

The distal sections and proximal sections may have different geometrical cross-sections. This may e.g. be such that the proximal sections' cross-section are hollow forming a hollow tubing so that connections to the sensor may be arranged inside said tubing. The proximal sections have then preferably a circular cross-section where the hollow tubing also has a circular cross-section which may be concentric in relation to the outer circular periphery.

### Example

Five different core wire designs were tested. All of the tested core wires had the general design shown in figure 5, but with different diameters for the sections B, C, F and G. For all of the tested core wires, the following dimensions apply:
LB=LF=2.5 mm
LC=0.40 mm
LD=1.85 mm dD=dE=0.275 mm
LE=0.55 mm

In Table 1 and Table 2 below, the dimensions, which were specific for the different core wire designs, are given together with the average number of strokes until break. Table 1 shows the results for core wires provided by a first supplier and Table 2 the results for core wires from a second supplier. The number of tested core wires for each design varied between five (5) and ten (10).

**Table 1:**

| *The number of strokes before break for different core wire designs for a first supplier of core wires.* | | | | | |
|---|---|---|---|---|---|
| Design | dB [mm] | dC [mm] | dF [mm] | dG[mm] | No.Strokes |
| I | 0.11 | 0.13 | 0.09 | 0.09 | 264 |
| II | 0.11 | 0.13 | 0.12 | 0.11 | 875 |
| III | 0.11 | 0.13 | 0.12 | 0.09 | 515 |
| IV | 0.09 | 0.11 | 0.11 | 0.09 | 1530 |
| V | 0.11 | 0.11 | 0.11 | 0.09 | 910 |

**Table 2:**

| *The number of strokes before break for different core wire designs for a second supplier of core wires.* | | | | | |
|---|---|---|---|---|---|
| Design | dB [mm] | dC [mm] | d [mm]F | d [mm]G | No.Strokes |
| I | 0.11 | 0.13 | 0.09 | 0.09 | 326 |
| II Not available | | | | | |
| III | 0.11 | 0.13 | 0.12 | 0.09 | 1090 |
| IV | 0.09 | 0.11 | 0.11 | 0.09 | 2305 |
| V | 0.11 | 0.11 | 0.11 | 0.09 | 1450 |

As can be seen from Table 1 and Table 2, for an optimal design regarding the strength of a core wire, the core wire should be as symmetrical as possible around the enlarged portion that is going to accommodate the sensor element. In particular, it is important that diameters of the portions adjacent to the enlarged portion are equal, i.e. dC should be equal to dF. Furthermore, for an optimal design of a core wire, the core wire should be symmetric around the enlarged portion, which here means that dC should be equal to dF and dB should be equal to dG.

## Claims

1. Core wire for a sensor guide wire assembly for intravascular measurements of physiological variables in a living body, the core wire (20, 30, 40) is provided with different longitudinal sections and comprises a mechanically rigid portion (C, D, E, 42), where a sensor is adapted to be arranged, one or many distal sections positioned distally said rigid portion and one or many proximal sections positioned proximally said rigid portion, **characterized in that** the ratio between the bending resistance of the sections adjacent said rigid portion is close to 1, preferably between 0,95 and 1,05.

2. Core wire according to claim 1, **characterized, in that** said longitudinal sections each having a section diameter (dA-dG), and that said mechanically rigid portion is a mechanically rigid enlarged portion that includes a predetermined number of sections (C, D, E) having larger diameters compared to the diameters of the distal (F, G) and proximal (A, B) sections, wherein the ratio between the diameters (dF, dC) adjacent the enlarged portion is close to 1, preferably between 0,95 and 1,05.

3. Core wire according to claim 2, **characterized in that** the relative absolute value difference between the diameters of the sections closest the enlarged portion is less than 5%.

4. Core wire according to claim 2, **characterized in that** diameters of the core wire adjacent to the enlarged portion are equal, i.e. dC equals dF.

5. Core wire according to claim 2, **characterized in that** the core wire is symmetrically shaped distally and proximally the enlarged portion.

6. Core wire according to claim 2, **characterized in that** the core wire is symmetrical around the enlarged portion with regard to the diameters of the core wire such that dC equals dF and dB equals dG.

7. Core wire according to claim 2, **characterized in that** sections E and C are conically shaped.

8. Core wire according to claim 2, **characterized in that** the diameter in sections E and C increases continuously step-wise.

9. Core wire according to claim 2, **characterized in that** the dC and dF is in the interval 90-130 µm.

10. Core wire according to claim 2, **characterized in that** the largest diameter of the enlarged section is 250 - 400 µm.

11. Core wire according to claim 1, **characterized in that** the diameters of the sections adjacent said rigid portion are approximately the same as the diameter of the rigid portion.

12. Core wire according to claim 1, **characterized in that** the rigid portion is made from a different material compared to that of the proximal and distal sections.

13. Core wire according to any preceding claim, **characterized in that** the core wire is made of a metal, such as stainless steel, or a superelastic metal, e.g. Nitinol®.

14. Core wire according to any of claims 1-11, **characterized in that** the distal sections are made from one material and the proximal section are made from another material.

15. Core wire according to any of claims 1-11, **characterized in that** the distal sections are made from nitinol and the proximal sections are made from stainless steel.

16. Core wire according to any preceding claim, **characterized in that** parts of said core wire is heat-treated in order to achieve the required performance.

17. Core wire according to any preceding claim, **characterized in that** said distal sections are made from a combination of materials, preferably arranged such that a base material is covered by another material.

18. Core wire according to any preceding claim, **characterized in that** said distal sections and proximal sections have different geometrical cross-sections.

19. Core wire according to any preceding claim, **characterized in that** said proximal cross-section is hollow such that connections to the sensor may be arranged inside and along said sections.

20. Sensor guide wire assembly for intravascular measurements of physiological variables in a living body comprising a sensor element, an electronic unit, a signal transmitting cable connecting the sensor element to the electronic unit, a flexible tube having the cable and the sensor element disposed therein, **characterized in that** said assembly further comprises a core wire according to any of claims 1-19 that is disposed inside the sensor guide and extends along the entire length of the sensor guide wire inside the flexible tube.

21. Sensor guide wire assembly according to claim 20 when dependent on claim 1, **characterized in that** a first coil (55) is attached to the distal end of the enlarged portion.

22. Sensor guide wire assembly according to claim 21,**characterized in that** said assembly further comprises a second coil (56) attached to the proximal end of the enlarged portion.

## Patentansprüche

1. Kerndraht für eine Sensorführungsdraht-Anordnung zum intravaskulären Messen physiologischer Variablen in einem lebenden Körper, wobei der Kerndraht (20, 30, 40) mit unterschiedlichen Längsabschnitten ausgestattet ist und einen mechanisch starren Bereich (C, D, E, 42) enthält, in welchem ein Sensor angeordnet sein kann, wobei ein oder viele entfernte Abschnitte entfernt von dem starren Bereich und ein oder viele nahe Abschnitte nahe dem starren Bereich angeordnet sind, **dadurch gekennzeichnet, dass** das Verhältnis zwischen dem Biegewiderstand der dem starren Bereich benachbarten Abschnitte nahe bei 1 liegt, vorzugsweise zwischen 0,95 und 1,05.

2. Kerndraht nach Anspruch 1, **dadurch gekennzeichnet, dass** die Längsabschnitte jeweils einen Abschnittsdurchmesse (dA-dG) haben, und dass der mechanisch starre Bereich ein mechanisch starr vergrößerter Bereich ist, welcher eine vorbestimmte Anzahl an Abschnitten (C, D, E) enthält, welche im Vergleich zu den Durchmessern der entfernten (F, G) und nahen (A, B) Abschnitte größere Durchmesser haben, wobei das Verhältnis zwischen den Durchmessern (dF, dC) benachbart dem vergrößerten Bereich nahe bei 1 liegt, vorzugsweise zwischen 0,95 und 1,05.

3. Kerndraht nach Anspruch 2, **dadurch gekennzeichnet, dass** die relative absolute Wertdifferenz zwischen den Durchmessern der Abschnitte, welche sich am nächsten zu den vergrößerten Bereichen befinden, weniger als 5% beträgt.

4. Kerndraht nach Anspruch 2, **dadurch gekennzeichnet, dass** die Durchmesser des Kerndrahtes benachbart dem vergrößerten Bereich gleich gross sind, d. h. dC gleich dF ist.

5. Kerndraht nach Anspruch 2, **dadurch gekennzeichnet, dass** der Kerndraht entfernt und nahe dem vergrößerten Bereich symmetrisch geformt ist.

6. Kerndraht nach Anspruch 2, **dadurch gekennzeichnet, dass** der Kerndraht um den vergrößerten Bereich herum mit Bezug auf die Durchmesser des Kerndrahtes symmetrisch ist, so dass dC gleich dF und dB gleich dG sind.

7. Kerndraht nach Anspruch 2, **dadurch gekennzeichnet, dass** die Abschnitte E und C konisch geformt sind.

8. Kerndraht nach Anspruch 2, **dadurch gekennzeichnet, dass** der Durchmesser in den Abschnitten E und C ständig schrittweise zunimmt.

9. Kerndraht nach Anspruch 2, **dadurch gekennzeichnet, dass** der dC und der dF im Bereich 90 - 130 µm liegen.

10. Kerndraht nach Anspruch 2, **dadurch gekennzeichnet, dass** der größte Durchmesser des vergrößerten Abschnittes 250 - 400 µm beträgt.

11. Kerndraht nach Anspruch 1, **dadurch gekennzeichnet, dass** die Durchmesser der Abschnitte benachbart dem starren Bereich ungefähr gleich dem Durchmesser des starren Bereiches sind.

12. Kerndraht nach Anspruch 1, **dadurch gekennzeichnet, dass** der starre Bereich aus einem anderen Material hergestellt ist als die nahen und entfernten Abschnitte.

13. Kerndraht nach einem der vorhergehenden Ansprüche, **dadurch gekennzeichnet, dass** der Kerndraht aus einem Metall, wie z. B. rostfreiem Stahl, oder einem superelastischen Metall, z. B. Nitinol®, hergestellt ist.

14. Kerndraht nach einem der Ansprüche 1 - 11, **dadurch gekennzeichnet, dass** die entfernten Abschnitte aus einem Material und die nahen Abschnitte aus einem anderen Material hergestellt sind.

15. Kerndraht nach einem der Ansprüche 1 - 11, **dadurch gekennzeichnet, dass** die entfernten Abschnitte aus Nitinol und die nahen Abschnitte aus rostfreiem Stahl hergestellt sind.

16. Kerndraht nach einem der vorhergehenden Ansprüche, **dadurch gekennzeichnet, dass** Teile des Kerndrahtes hitzebehandelt sind, um die benötigte Leistung zu erzielen.

17. Kerndraht nach einem der vorhergehenden Ansprüche, **dadurch gekennzeichnet, dass** die entfernten Abschnitte aus einer Kombination von Materialien hergestellt sind, welche vorzugsweise so angeordnet sind, dass ein Basismaterial von einem anderen Material bedeckt ist.

18. Kerndraht nach einem der vorhergehenden Ansprüche, **dadurch gekennzeichnet, dass** die entfernten und nahen Abschnitte unterschiedliche geometrische Querschnitte aufweisen.

19. Kerndraht nach einem der vorhergehenden Ansprüche, **dadurch gekennzeichnet, dass** der nahe Querabschnitt hohl ist, so dass Verbindungen mit dem Sensor innen und entlang dieser Abschnitte angeordnet sein können.

20. Sensor-Führungsdraht-Anordnung zum intravaskulären Messen von physiologischen Variablen in einem lebenden Körper, enthaltend ein Sensorelement, eine elektronische Einheit, ein Signalübertragungskabel, welches das Sensorelement mit der elektronischen Einheit verbindet, ein flexibles Rohr enthaltend das Kabel und das darin angeordnete Sensorelement, **dadurch gekennzeichnet, dass** die Anordnung weiterhin einen Kerndraht gemäß einem der Ansprüche 1 - 19 enthält, welcher in der Sensorführung gelegen ist und sich längs der gesamten Länge des Sensorführungsdrahtes in dem flexiblen Rohr befindet.

21. Sensor-Führungsdraht-Anordnung nach Anspruch 20, wenn abhängig von Anspruch 1, **dadurch gekennzeichnet, dass** eine erste Spule (55) an dem entfernten Ende des vergrößerten Bereiches angebracht ist.

22. Sensor-Führungsdraht-Anordnung nach Anspruch 21, **dadurch gekennzeichnet, dass** die Anordnung weiterhin eine zweite Spule (56) enthält, welche an dem entfernten Ende des vergrößerten Bereiches angebracht ist.

## Revendications

1. Fil métallique central destiné à un ensemble de fil métallique de guidage de capteur servant à des mesures intra-vasculaires des variables physiologiques d'un corps vivant, le fil métallique central (20, 30, 40) étant doté de différentes sections longitudinales et comprenant une partie rigide mécaniquement (C, D, E 42), dans laquelle un capteur est adapté pour y être disposé, une ou plusieurs section(s) distale(s) étant positionnée(s) de façon distale par rapport à ladite partie rigide et une ou plusieurs sections proximales étant positionnées de façon proximale par rapport à ladite partie rigide, **caractérisé en ce que** le rapport entre la résistance à la courbure des sections adjacentes à ladite partie rigide est voisin de 1, de préférence est compris entre 0,95 et 1,05.

2. Fil métallique central selon la revendication 1, **caractérisé en ce que** lesdites sections longitudinales présentent, chacune, un diamètre de section (dA-dG) et **en ce que** ladite partie rigide mécaniquement est une partie agrandie rigide mécaniquement qui comporte un nombre prédéterminé de sections (C, D, E) présentant des diamètres plus grands comparés aux diamètres des sections distales (F,G) et proximales (A,B), dans lequel le rapport entre les diamètres (dF, dC) adjacents à la partie agrandie est voisin de 1, de préférence est compris entre 0,95 et 1,05.

3. Fil métallique central selon la revendication 2, **caractérisé en ce que** la différence de valeur absolue relative entre les diamètres des sections les plus proches de la partie agrandie est inférieur à 5%.

4. Fil métallique central selon la revendication 2, **caractérisé en ce que** les diamètres du fil métallique central adjacent à la partie agrandie sont égaux, c'est à dire que dC est égal à dF.

5. Fil métallique central selon la revendication 2, **caractérisé en ce que** le fil métallique central est configuré symétriquement de façon distale et de façon proximale par rapport à la partie agrandie.

6. Fil métallique central selon la revendication 2, **caractérisé en ce que** le fil métallique central est symétrique autour de la partie agrandie en ce qui concerne les diamètres du fil métallique central de sorte que dC est égal à dF et que dB est égal à dG.

7. Fil métallique central selon la revendication 2, **caractérisé en ce que** les sections E et C sont configurées de façon conique.

8. Fil métallique central selon la revendication 2, **caractérisé en ce que** le diamètre des sections E et C augmente par étapes, de façon continue.

9. Fil métallique central selon la revendication 2, **caractérisé en ce que** les diamètres dC et dF se situent dans l'intervalle de 90 à 130 µm.

10. Fil métallique central selon la revendication 2, **caractérisé en ce que** le diamètre le plus grand de la section agrandie est de 250 à 400 µm.

11. Fil métallique central selon la revendication 1, **caractérisé en ce que** les diamètres des sections adjacentes à ladite partie rigide sont approximativement les mêmes que le diamètre de la partie rigide.

12. Fil métallique central selon la revendication 1, **caractérisé en ce que** la partie rigide est constituée d'un matériau différent comparé à celui des sections distales et proximales.

13. Fil métallique central selon l'une quelconque des revendications précédentes, **caractérisé en ce que** le fil métallique central est constitué d'un métal, tel que l'acier inoxydable, ou d'un métal super élastique, par exemple, le Nitinol®.

14. Fil métallique central selon l'une quelconque des revendications 1 à 11, **caractérisé en ce que** les sections distales sont constituées d'un matériau et **en ce que** les sections proximales sont constituées d'un autre matériau.

15. Fil métallique central selon l'une quelconque des revendications 1 à 11, **caractérisé en ce que** les sections distales sont constituées de nitinol et **en ce que** les sections proximales sont constituées d'acier inoxydable.

16. Fil métallique central selon l'une quelconque des revendications précédentes, **caractérisé en ce que** des parties dudit fil métallique central sont traitées thermiquement afin d'obtenir la performance requise.

17. Fil métallique central selon l'une quelconque des revendications précédentes, **caractérisé en ce que** lesdites sections distales sont constituées d'une combinaison de matériaux, de préférence, disposé de façon qu'un matériau de base soit recouvert par un autre matériau.

18. Fil métallique central selon l'une quelconque des revendications précédentes, **caractérisé en ce que** lesdites sections distales et les dites sections proximales présentent des sections transversales géométriques différentes.

19. Fil métallique central selon l'une quelconque des revendications précédentes, **caractérisé en ce que** ladite section transversale proximale est creuse de telle sorte que des connexions vers le capteur peuvent être disposées à l'intérieur et le long desdites sections.

20. Ensemble de fil métallique de guidage de capteur destiné à des mesures intra-vasculaires des variables physiologiques d'un corps vivant comportant un élément de capteur, une unité électronique, un câble de transmission de signal connectant l'élément de capteur à l'unité électronique, un tube souple contenant en lui le câble et l'élément de capteur, **caractérisé en ce que** ledit ensemble comprend, de plus, un fil métallique central selon l'une quelconque des revendications 1 à 19 qui est disposé à l'intérieur du guide de capteur et qui s'étend sur toute la longueur du fil métallique de guidage de capteur à l'intérieur du tube souple.

21. Ensemble de fil métallique de guidage de capteur selon la revendication 20 lorsque dépendante de la revendication 1, **caractérisé en ce qu'**une première bobine (55) est fixée à l'extrémité distale de la partie agrandie.

22. Ensemble de fil métallique de guidage de capteur selon la revendication 21, **caractérisé en ce que** ledit ensemble comprend, de plus, une seconde bobine (56) fixée à l'extrémité proximale de la partie agrandie.
